# EUROPEAN PATENT SPECIFICATION

(11) **EP 1 363 689 B1**
(45) Date of publication and mention of the grant of the patent: **25.10.2006**
(21) Application number: 02701841.5
(22) Date of filing: 28.02.2002
(51) Int. Cl.: A61M 5/24, A61M 5/28, A61M 5/315

(54) **INJECTION SYRINGE HAVING DOSAGE DEVICE**
INJEKTIONSSPRITZE MIT DOSIERVORRICHTUNG
SERINGUE A INJECTION POURVUE D'UN DOSEUR

(30) Priority: 28.02.2001 SE 0100709
(43) Date of publication of application: 26.11.2003
(73) Proprietor: Mats Bonnier Sjukvard Och Utveckling AB, 427 36 Billdal (SE)
(72) Inventor: BONNIER, Mats, 427 36 Billdal (SE)
(74) Representative: Inger, Lars Ulf Bosson
(86) International application number: PCT/SE2002/000353
(87) International publication number: WO 2002/070050

(56) References cited:
- EP-A2- 0 937 476
- US-A- 5 092 842
- US-A- 5 295 976
- US-A- 5 454 793
- US-A- 5 688 250
- US-A- 6 059 755

## Description

### TECHNICAL AREA

A hypodermic syringe comprising an outer syringe being arranged in connection with an ampoule for medical agent. The ampoule is mutually displaceable inside the outer syringe, whereby an indication device is arranged to the ampoule and applied to be uncovered at the mutual displacement, whereby a dosage is adjustable and readable. In particular the invention relates to an injection device comprising an outer syringe with a dose indication device which is arranged in connection with an ampoule for medical agent comprising a piston inserted therein, the ampoule is axially displaceable inside the outer syringe relatively to this.

### BACKGROUND OF THE INVENTION

Diabetes is a chronic metabolic disease, which is characterised in the lack of the hormone insulin. There is different types of diabetes, whereof the type 1 diabetes exists when the ability of the pancreas to produce insulin successively decrease or cease, leading to lack of insulin. At the type 1 diabetes the treatment, except for cost treatment, also consists of supplying insulin, which is given via injection under the skin four to eight times a day.

Diabetes is a disease, which in worst case, particularly at the type 1 diabetes, may cause death if not treated in a correct way. In other cases complications may appear such as kidney injuries, lack of circulation, particularly the extremities, eye damages leading to blindness, to early death, etcetera.

Technical means exist such as syringe pencils and insulin pumps, considerably facilitating the supply of insulin. The syringe pencil contains insulin and a hypodermic needle in one unit, which, for instance, can be carried in the breast pocket. Insulin pumps are battery operated and carried outside the body, and then the insulin is introduced via a plastic catheter and a hypodermic needle being mounted under the skin.

In the present-day situation it is very common that the diabetic patient him or herself injects the insulin by means of a syringe pencil according to the construction mentioned above. When a syringe pencil is carried in the daily life it is desirable that it is easy to use, reliable, as accurate as possible in dosing. Another desired object is that one and the same syringe pencil can be used several times, even having the same medicine ampoule left therein.

The state of the art shows a number of syringe pencils.

In EP 0 688 571 and EP 0 702 970 a medicine pencil with an injection and dosage construction is shown, having a very complex construction with a large number of parts. In EP 0 388 806 a syringe exists, which comprises a.o. a helical spring with a rotary casing for dosage.

Additional syringe pencils are shown in WO 89/07463 and DE 40 13 769, which, however, exhibit very complex constructions.

US-A-5,454,793 shows an injection syringe having a rotating, piston rod-receiving unit, in which unit an ampoule can be inserted and rotated into position in the syringe before administrating. There is not any possibility to predetermine the dose size in this device.

US-A-5,295,976 discloses an injection device comprising an outer syringe which is arranged in connection with an ampoule with a piston therein. The ampoule is thereby arranged to be moved axially in relation to the syringe via a thread system. The syringe further encompasses a dose indication device arranged on the top of the syringe and being connected to the rotation of the periphery of the thread system.

Altogether, the documents above indicates that improvements of syringe pencils are desirable in the aspect of achieving syringe pencils having a simple construction, being inexpensive to produce, and which are also appropriate for use several times having the same ampoule or cartridge containing medical agent, or reusable with the exchange of the medical ampoule or cartridge.

### SUMMARY OF THE INVENTION

The main object of the present invention is to provide an injection device according to the preamble of claim 1 comprising an outer syringe, which is arranged in connection with an ampoule for medical agent. The ampoule is mutually movable or displaceable inside the outer syringe, whereby an indication device is arranged in connection with the ampoule, which is applied to be uncovered at the mutual displacement, whereby a dosage can be adjustable and readable.

This object is obtained by means of a device more precisely defined in the characterizing clause of claim 1. The preferred embodiments are evident by the appended claims.

### SHORT DESCRIPTION OF THE DRAWINGS

In the following, the invention will be described in more detail and in a non-limiting way with reference to the accompanying drawings, in which:
- Fig. 1a: schematically shows a side-view of a piston to an injection pencil;
- Fig. 1b: schematically shows a side-view of an ampoule or cartridge containing medical agent to an injection pencil;
- Fig. 1c: schematically shows a side-view of an outer syringe according to a first and a second preferred embodiment of the invention;
- Fig. 1d: schematically shows a side-view of an outer front part of an outer syringe according to a second preferred embodiment of the invention;
- Fig. le: schematically shows a side-view and a front-view of a supporting washer to an injection pencil of a preferred embodiment of the invention ;
- Fig. 1f: schematically shows a side-view of a safety casing for the hypodermic needle of an injection pencil;
- Fig. 1g: schematically shows a side-view of a safety plug for the piston to an injection pencil;
- Fig. 2: schematically shows a side-view of the hypodermic needle and the piston arranged in connection with a medical ampoule or cartridge containing medical agent;
- Fig.3: schematically shows a side-view of the hypodermic needle, the piston and the medical ampoule or cartridge containing the medical agent arranged in an outer syringe according to a first embodiment of the invention; and
- Fig. 4: schematically shows a side-view of the hypodermic needle, the piston and the medical ampoule or cartridge containing the medical agent arranged in an outer syringe according to a second embodiment of the invention.

### DETAILED DESCRIPTION OF EMBODIMENTS

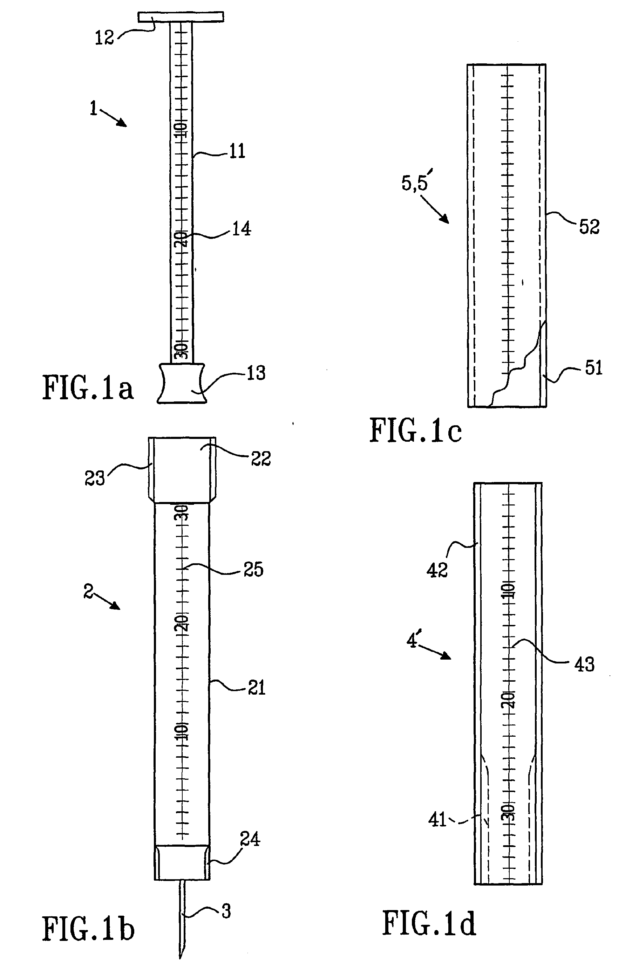

An injection device with a dosage device comprises a piston 1, an ampoule 2, a hypodermic syringe 3, a first preferred embodiment of an outer syringe 5, or an outer front member of a second preferred injection device 4'and a second preferred embodiment of an outer syringe 5', a support washer 6, a protecting cover 7 and a safety plug 8, see Figs. 1a -g.

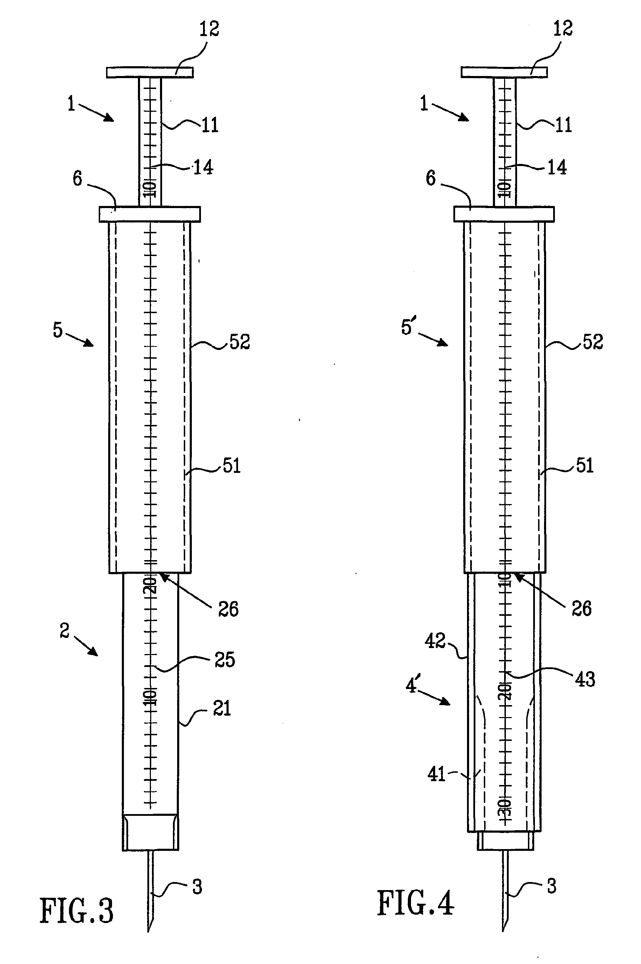

The injection device such as a syringe pencil is in a first preferred embodiment particularly preferred for use one or several times, see Fig. 3. A second preferred embodiment of the syringe pencil is particularly preferred for use several times and reuse, see Fig. 4. With reuse meansthat it is intended for one or a number of exchanges of the ampoule 2 of the syringe pencil to new ampoules 2 containing medical.

In a preferred embodiment of the invention said ampoule 2 is preferably a container such as an ampoule or a cartridge containing a medical agent in multiple production in the present-day-situation. A hypodermic needle 3 is fixedly arranged to the ampoule 2, see Fig. 1b. Moreover, a piston 1 is arranged in connection with the ampoule 2.

In greater detail, said piston 1 comprises a rod piston 11, a tube or the like, preferably with a substantially circular cross section. The piston 1 is in one of its ends provided with a washer 12, see Fig. 1a, which is used as a pressure surface at injection, e.g. by a thumb of a patient. The piston 1 is in its other end provided with a sealing device 13. The sealing device 13 is preferably a sealing body of an elastic material, e.g. a polymer, a rubber or the like, which closes tightly at contact and abutment to a surface. Such sealing and sealing materials are known at prevalent syringes.

In a preferred embodiment of the invention the piston 1 is provided with an indication device 14, preferably in the form of a graduated longitudinal scale, which is divided into units, which preferably correspond to volume units of medical agent.

Further the ampoule 2 is shaped as an elongated hollow tube 21, being closed at both ends, and closes a medical agent in liquid form, see Fig. 1b. The ampoule 2 preferably shows a substantially circular cross section. The ampoule 2 is provided with a stopping device 22 in one of its ends, e.g. a lip, wedge, collar or the like, which for instance shows a stop position for the piston 1 at the injection by the ampoule 2. The ampoule 2 is provided with a fixedly arranged hypodermic needle 3 in its other end.

In a first particularly preferred embodiment of the invention according to above, the ampoule 2 is provided with a connection device 23 for connecting the ampoule 2 with the outer syringe 5, see Figs 1b-c. The connection device 23 is preferably arranged in the same end of the ampoule 2 as the stopping device 22. The connection device 23 consists preferably of external threads, splines or the like.

In a second particularly preferred embodiment of the invention according to above the ampoule is provided with a second connection device 24 for connecting the ampoule 2 to a second embodiment of the injection device 5'. The connection device 24 is preferably arranged in connection with the hypodermic needle 3, and consists preferably of external threads, splines or the like.

Finally, the ampoule 2 is provided with an indication device 24 or reading device on its surrounding surface or envelope surface, preferably in the form of a graduated longitudinal scale. The indication device 25 is divided into units corresponding to volume unit's medical agent in the ampoule 2.

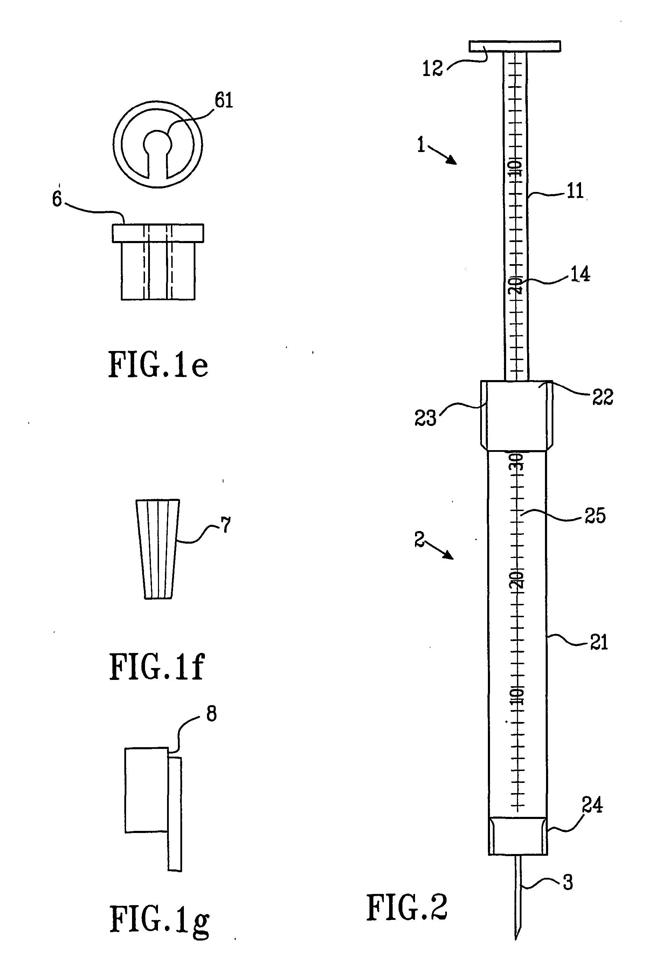

Altogether, concerning the medicine ampoule 2 in its entirety, said ampoule 2 is mutually arranged with the hypodermic needle 3 and the piston 1 according to the following. The sealing device 13 and a part of the piston rod 11 is applied inside the ampoule 2 in such a way that the sealing device 13 fits tightly against the interior enveloping surface of the ampoule 2, see Fig.2. The sealing device 13 provides that leakage of the medical agent does not occur of the ampoule 2, and particularly not in connection with the sealing device 13. Further, the sealing device 13 can slide along the interior enveloping surface of the ampoule 2 with the movement of the piston 1 inside the ampoule 2, preferably in the direction toward the hypodermic needle 3, whereby the sealing device 13 fits tightly against the interior enveloping surface without leakage of the medical agent occuring from the ampoule 2.

In a first preferred embodiment of the invention, the ampoule 2, the hypodermic needle 3 and the piston 1, which are mutually arranged according the description above, are arranged in said outer syringe 5, see Fig. 3.

The outer syringe 5 comprises a part, such as a tube 52, housing or the like, see Fig. 1c. The outer syringe 5 shows a substantially circular cross section and an inside diameter being substantially larger than the outer diameter of the ampoule 2. Further, the outer syringe 5 is provided with a connection device 51 for connection with the ampoule 2.The connection device 51 is preferably arranged along the interior surrounding surface or the enveloping surface of the outer syringe 5, and preferably comprises internal threads, splines or the like, corresponding and co-operating with the connection device 23 of the ampoule 2.

At the connection of the ampoule, comprising the hypodermic needle 3 and the piston 1, with the outer syringe 5, the ampoule is fastened into the outer syringe 5 by means of the connection devices 23 and 51. In this way, the ampoule 2 is also moveable or displaceable inside the outer syringe 5, whereby the outer syringe is moveable along the outer envelope surface of the ampoule 2 in a corresponding way, see Fig.3.

The outer syringe 5 is provided with a supporting washer 6 for achieving an improved axial operation of the movement of the piston 1 inside the ampoule 2. The supporting washer 6 comprises a spline 61, such as a hole, recess or the like, in which spline 61 the piston rod 11 of the piston 1 is arranged, see Fig. 1e. Consequently, the supporting washer 6 achieves an improved accuracy and reliability, and decreases the risk for leakage of the injection pencil as well.

The injection pencil according a first improved embodiment is used in the following way.

A dosage is performed by means of the relative displacement of the outer syringe 5 along the ampoule 2 in the direction toward the hypodermic needle 3 before the injection, which is applicable both for use of a new ampoule 2 and the succeeding injections. Thus, an adjustment of the specific medicine dose is carried out by means of the required number of units or scale shares according to the indication device 25.

In a preferred embodiment of the invention, a reading of the position of the outer syringe 5 relative to the ampoule 2 occurs, for instance in the uncovered point 26 between the outer syringe 5 and the ampoule 2 of the indication device 25, see Fig. 3.

At the injection the outer syringe 5 is screwed, in the direction towards the hypodermic needle 3, the number of scale units of medical agent corresponding to the medicine dose needed according to the indication device 25. Then the piston 1 is pushed into the ampoule 2 by pressing the washer 12. At the movement of the piston 1 into the ampoule 2 the medical agent is pressed out of the ampoule 2 via the hypodermic needle 3. The piston 1 is stopped in its movement by the supporting washer 6, whereby the injection according to the adjusted dose of medical agent is executed.

According to the description mentioned above dosage and injection can be repeated until the piston 1 substantially reaches the bottom or the end position of the ampoule 2, whereby the ampoule 2 has been emptied of medical agent.

The remaining amount of medical agent of the ampoule 2 substantially corresponds to the absolute position of the outer syringe 5 along the ampoule 2, specified in units or scale shares of the indication device 25, which is applicable after the previous injection, but on the other hand before dosage.

When there is an emptied medicine ampoule 2 in the injection pencil an exchange to a new ampoule 2 is performed by demounting the supporting washer 6 before the empty ampoule 2 is screwed out of the outer syringe 5, preferably in the opposite direction than at dosage, by means of the connection devices 51 and 23. A new ampoule 2 containing medical agent is arranged to the outer pencil 5 according to above.

Finally, the injection pencil is provided with a protecting cover 7 for protecting the patient against the needlepoint of the hypodermic needle 3 at bringing the injection pencil, but also for giving a substantially sterile needlepoint of the hypodermic needle 3, see Fig. 1f. Moreover, the injection pencil is provided with a safety plug for avoiding that the piston is exposed for unnecessary pushs or impacts, see Fig. 1g.

A second particularly preferred embodiment of the invention comprises an outer syringe 5'and an outer front part 4'for the outer syringe 5', except the included parts of the injection device mentioned above, see Fig. 4.

According to a second particularly preferred embodiment of the invention, the outer syringe 5'has substantially the same construction as in the said first preferred embodiment, except regarding dimensions, etc.

The said outer front part of the outer syringe 4'is a tube, a casing or the like having a substantially circularly cross section and a diameter substantially larger than the diameter of said ampoule 2, see Fig. 1d. Further, the member 4' s provided with an connection device 41 for the ampoule 2is in one of its internal end, preferably in form of internal threads, splines or the like, and provided with an connection device 42 for the outer syringe 5' on its outer surrounding surface or envelope surface, preferably in the form of external threads, splines or the like.

Moreover, the outer front part 4'to the outer syringe 5'is provided with an indication device 43 on its surrounding outer surface or enveloping surface, preferably in the form of a longitudinal readable scale, in the corresponding way as the ampoule 2 is provided with an indication device 23 according to the first particularly preferred embodiment of the invention.

The outer syringe 5'exhibits a diameter being substantially larger than the diameter of the outer front part 4'of the outer syringe 5'and an inside diameter adjusted to the outer diameter of the front part. The connection device 51 of the outer syringe 5'corresponds to and co-operates with the outer connection device 42 of the outer front part 4'of the outer syringe 5'in the same way as the connection devices 51 and 23 are corresponding and co-operating between the outer syringe 5 and the ampoule 2 according to the first particularly preferred embodiment of the invention.

In a second particularly preferred embodiment of the invention, the ampoule 2 is brought inside the outer front part of the outer syringe 4'by inserting and screwing the ampoule 2 into the outer front part 4', whereby this is brought to an end position, preferably to a locking by means of the connection devices 41 and 24. Then the outer front part 4'is brought to the outer syringe 5' comprising the ampoule 2, preferably by insertion and displacement by means of the connection devices 51 and 42, respectively, see Fig. 4.

Thus, the outer syringe 5'is displaceable along the outer front part 4'in a direction toward the hypodermic needle 3, whereby the outer pencil 5' is displaceable along the indication device 42 in the corresponding way as the outer syringe 5 according to the first preferred embodiment is displaceable or telescopeable along the indications device 23.

Dosage and injection with a hypodermic syringe, according the second particularly preferred embodiment of the invention, is carried out in a corresponding way as with the injection pencil according to the first preferred embodiment of the invention.

The outer syringe 5, 5'is also provided with an indication device 53 according to other embodiments of the invention.

In a particularly preferred embodiment said injection pencil is an insulin pencil, and said medical agent is consequently insulin.

The invention is not limited to the shown embodiments but can be varied in a number of different ways, for instance by combination of two or more of the embodiments shown, without depart from the scope of the appended claims, and the arrangement and the method can be implemented in a number of ways depending on application, functional units, needs and requirements and so on.

## Claims

1. An injection device comprising a dose indication device and an outer syringe (5, 5') which is arranged in connection with an ampoule (2) for medical agent comprising a piston inserted therein, the ampoule (2) is axially displaceable inside the outer syringe (5, 5') relatively to this,
**characterised in**
**that** an indication device (14, 25, 43) is arranged in connection with the ampoule (2), which is established to be uncovered at the mutual displacement, whereby a dosage is adjustable and readable by the displacement of the ampoule relatively to the outer syringe (5, 5'), and whereby the piston rod is arranged to cooperate with the outer syringe (5, 5') for giving said dose.

2. An injection device as claimed in claim 1,
**characterised in**
**that** the outer syringe (5) is provided with a connection device (51) in form of threads, splines or the like, and that the ampoule (2) is provided with a connection device (23) in form of threads, splines or the like, whereby these connection devices (51, 23) cooperate with each other.

3. An injection device as claimed in claim 2,
**characterised in**
**that** the ampoule (2) is mutually displaceable inside the outer syringe (5) by means of said connection devices (51, 23).

4. An injection device as claimed in any of the proceeding claims,
**characterised in**
**that** the ampoule (2) is preferably an ampoule or cartridge, containing a medical agent, existing in multiple production in the present-day-situation.

5. An injection device as claimed in any of the proceeding claims,
**characterised in**
**that** a piston (1) and a hypodermic needle (3) are arranged in connection with said ampoule (2).

6. An injection device as claimed in any of the proceeding claims,
**characterised in**
**that** a indication device (25) is arranged on the enveloping surface of the ampoule (2), preferably in the form of a graduated longitudinal scale, which is divided into units corresponding to volume units of medical agent.

7. An injection device as claimed in claim 1,
**characterised in**
**that** the outer syringe (5') is provided with a connection device (51) in the form of threads, splines or the like, and a front part arranged to the outer syringe (4') is provided with a connection device (42) in the form of threads, splines or the like, whereby these connection devices (51, 42) cooperate with each other.

8. An injection device as claimed in claim 7,
**characterised in**
**that** the outer front part (4') comprising the ampoule (2) is mutually displaceable inside the outer syringe (5') by means of said connection devices (51, 42).

9. An injection device as claimed in any of the claims 7 to 8,
**characterised in**
**that** the ampoule (2) is provided with a connection device (24) in the form of threads, splines or the like, and the front forward part (4') is provided with a connection device (41) of an outer front part (4'), whereby these cooperate with each other.

10. An injection device as claimed in any of the claims 7 to 9,
**characterised in**
**that** the ampoule (2) is fixedly arranged to the outer front part (4') by means of the connection devices (24, 41).

11. An injection device as claimed in any of the claims 7 to 10,
**characterised in**
**that** a indication device (43) is arranged on the surrounding or enveloping surface of the outer front part (4'), preferably in the form of a graduated longitudinal scale, which is divided into units corresponding to volume units of medical agent.

12. An injection device according to any of the proceeding claims,
**characterised in**
**that** the injection device preferably shows a protecting cover for the hypodermic needle, and a safety plug for the piston.

## Patentansprüche

1. Injektionsvorrichtung, die eine Dosisangabebvorrichtung und eine äußere Spritze (5, 5'), die in Verbindung mit einer Ampulle (2) für ein medizinisches Mittel angeordnet ist und einen darin eingesetzten Kolben aufweist, umfasst, wobei die Ampulle (2) in der äußeren Spritze (5, 5') relativ hierzu axial verlagerbar ist,
**dadurch gekennzeichnet,**
**dass** in Verbindung mit der Ampulle (2) eine Anzeigevorrichtung (14, 25, 43) vorgesehen ist, die so beschaffen ist, dass sie bei der relativen Verlagerung aufgedeckt wird, wodurch eine Dosierung durch die Verlagerung der Ampulle relativ zu der äußeren Spritze (5, 5') einstellbar und ablesbar ist, wobei die Kolbenstange so beschaffen ist, dass sie mit der äußeren Spritze (5, 5') zusammenwirkt, um diese Dosis abzugeben.

2. Injektionsvorrichtung nach Anspruch 1,
**dadurch gekennzeichnet,**
**dass** die äußere Spritze (5) mit einer Verbindungsvorrichtung (51) in Form von Gewinden, Keilnuten oder dergleichen versehen ist und dass die Ampulle (2) mit einer Verbindungsvorrichtung (23) in Form von Gewinden, Keilnuten oder dergleichen versehen ist, so dass diese Verbindungsvorrichtungen (51, 23) zusammenwirken.

3. Injektionsvorrichtung nach Anspruch 2,
**dadurch gekennzeichnet,**
**dass** die Ampulle (2) in der äußeren Spritze (5) mittels der Verbindungsvorrichtungen (51, 23) hierzu verlagerbar ist.

4. Injektionsvorrichtung nach einem der vorhergehenden Ansprüche,
**dadurch gekennzeichnet,**
**dass** die Ampulle (2) vorzugsweise eine ein medizinisches Mittel enthaltende Ampulle oder Kassette ist, wie es sie in modernen Massenproduktionen gibt.

5. Injektionsvorrichtung nach einem der vorhergehenden Ansprüche,
**dadurch gekennzeichnet,**
**dass** in Verbindung mit der Ampulle (2) ein Kolben (1) und eine Subkutannadel (3) vorgesehen sind.

6. Injektionsvorrichtung nach einem der vorhergehenden Ansprüche,
**dadurch gekennzeichnet,**
**dass** auf der Hüllfläche der Ampulle (2) eine Anzeigevorrichtung (25) vorzugsweise in Form einer longitudinalen Skala, die in Einheiten unterteilt ist, die Volumeneinheiten des medizinischen Mittels entsprechen, vorgesehen ist.

7. Injektionsvorrichtung nach Anspruch 1,
**dadurch gekennzeichnet,**
**dass** die äußere Spritze (5') mit einer Verbindungsvorrichtung (51) in Form von Gewinden, Keilnuten oder dergleichen versehen ist und ein vorderer Teil, der an der äußeren Spritze (4') angeordnet ist, mit einer Verbindungsvorrichtung (42) in Form von Gewinden, Keilnuten oder dergleichen versehen ist, wodurch diese Verbindungsvorrichtungen (51, 42) zusammenwirken.

8. Injektionsvorrichtung nach Anspruch 7,
**dadurch gekennzeichnet,**
**dass** der äußere vordere Teil (4'), der die Ampulle (2) umfasst, in der äußeren Spritze (5') mittels der Verbindungsvorrichtungen (51, 42) hierzu verlagerbar ist.

9. Injektionsvorrichtung nach einem der Ansprüche 7 bis 8,
**dadurch gekennzeichnet,**
**dass** die Ampulle (2) mit einer Verbindungsvorrichtung (24) in Form von Gewinden, Keilnuten oder dergleichen versehen ist und dass der vordere Teil (4') mit einer Verbindungsvorrichtung (41) an einem äußeren vorderen Teil (4') versehen ist, wodurch diese zusammenwirken.

10. Injektionsvorrichtung nach einem der Ansprüche 7 bis 9,
**dadurch gekennzeichnet,**
**dass** die Ampulle (2) an dem äußeren vorderen Teil (4) mittels der Verbindungsvorrichtungen (24, 41) fest angeordnet Ist.

11. Injektionsvorrichtung nach einem der Ansprüche 7 bis 10,
**dadurch gekennzeichnet,**
**dass** eine Anzeigevorrichtung (43) an der umgebenden oder umhüllenden Oberfläche des äußeren vorderen Teils (4') vorzugsweise in Form einer longitudinalen Skala, die in Einheiten unterteilt ist, die Volumeneinheiten des medizinischen Mittels entsprechen, angeordnet ist.

12. Injektionsvorrichtung nach einem der vorhergehenden Ansprüche,
**dadurch gekennzeichnet,**
**dass** die Injektionsvorrichtung vorzugsweise eine Schutzabdeckung für die Subkutannadel und einen Sicherheitsstopfen für den Kolben umfasst.

## Revendications

1. Dispositif d'injection comportant un dispositif d'indication de dose et une seringue extérieure (5, 5') qui est agencée en connexion avec une ampoule (2) pour un agent médical comportant un piston inséré dans celle-ci, l'ampoule (2) pouvant être déplacée axialement à l'intérieur de la seringue extérieure (5, 5') relativement à celle-ci,
**caractérisé en ce qu'**un dispositif d'indication (14, 25, 43) est agencé en connexion avec l'ampoule (2), qui est établie pour être découverte lors du déplacement mutuel, de sorte qu'un dosage est ajustable et lisible par le déplacement de l'ampoule relativement à la seringue extérieure (5, 5'), et de sorte que la tige de piston est agencée pour coopérer avec la seringue extérieure (5, 5') pour délivrer ladite dose.

2. Dispositif d'injection selon la revendication 1,
**caractérisé en ce que** la seringue extérieure (5) est munie d'un dispositif de connexion (51) en forme de filets, cannelures ou analogues, et que l'ampoule (2) est munie d'un dispositif de connexion (23) sous la forme de filets, cannelures ou analogues, de sorte que ces dispositifs de connexion (51, 23) coopèrent l'un avec l'autre.

3. Dispositif d'injection selon la revendication 2,
**caractérisé en ce que** l'ampoule (2) peut être déplacée mutuellement à l'intérieur de la seringue extérieure (5) par l'intermédiaire desdits dispositifs de connexion (51, 23).

4. Dispositif d'injection selon l'une quelconque des revendications précédentes,
**caractérisé en ce que** l'ampoule (2) est de préférence une ampoule ou une cartouche, contenant un agent médical, existant en production multiple, dans la situation actuelle.

5. Dispositif d'injection selon l'une quelconque des revendications précédentes,
**caractérisé en ce qu'un** piston (1) et une aiguille hypodermique (3) sont agencés en connexion avec ladite ampoule (2).

6. Dispositif d'injection selon l'une quelconque des revendications précédentes,
**caractérisé en ce qu'**un dispositif d'indication (25) est agencé sur la surface enveloppante de l'ampoule (2), de préférence sous la forme d'une échelle longitudinale graduée, qui est divisée en unités correspondant à des unités de volume d'un agent médical.

7. Dispositif d'injection selon la revendication 1,
**caractérisé en ce que** la seringue extérieure (5') est munie d'un dispositif de connexion (51) sous la forme de filets, cannelures ou analogues, et une partie frontale agencée sur la seringue extérieure (4') est munie d'un dispositif de connexion (42) sous la forme de filets, cannelures ou analogues, de sorte que ces dispositifs de connexion (51, 42) coopèrent l'un avec l'autre.

8. Dispositif d'injection selon la revendication 7,
**caractérisé en ce que** la partie frontale extérieure (4') comportant l'ampoule (2) peut être déplacée mutuellement à l'intérieur de la seringue extérieure (5') par l'intermédiaire desdits dispositifs de connexion (51, 42).

9. Dispositif d'injection selon les revendications 7 et 8,
**caractérisé en ce que** l'ampoule (2) est munie d'un dispositif de connexion sous la forme de filets, cannelures ou analogues, et la partie frontale avant (4') est munie d'un dispositif de connexion (41) d'une partie frontale extérieure (4'), de sorte que celles-ci coopèrent l'une avec l'autre.

10. Dispositif d'injection selon l'une quelconque des revendications 7 à 9,
**caractérisé en ce que** l'ampoule (2) est agencée de manière fixe sur la partie frontale extérieure (4') par l'intermédiaire des dispositifs de connexion (24, 41).

11. Dispositif d'injection selon l'une quelconque des revendications précédentes,
**caractérisé en ce qu'**un dispositif d'indication (43) est agencé sur la surface entourante ou enveloppante de la partie frontale extérieure (4'), de préférence sous la forme d'une échelle longitudinale graduée qui est divisée en unités correspondant à des unités de volume d'un agent médical.

12. Dispositif d'injection selon l'une quelconque des revendications précédentes,
**caractérisé en ce que** le dispositif d'injection présente de préférence un recouvrement protecteur pour l'aiguille hypodermique, et un bouchon de sécurité pour le piston.
